# EUROPÄISCHE PATENTSCHRIFT

(11) **EP 1 935 355 B1**
(45) Veröffentlichungstag und Bekanntmachung des Hinweises auf die Patenterteilung: **02.12.2015**
(21) Anmeldenummer: 06025588.2
(22) Anmeldetag: 11.12.2006
(51) Int. Cl.: A61L 29/06, A61B 17/00, A61L 29/08, A61L 29/16, A61B 17/34, A61J 15/00

(54) **Schlauch für die enterale Ernährung**
Tube for enteral nutrition
Tuyau flexible pour l'alimentation entérale

(43) Veröffentlichungstag der Anmeldung: 25.06.2008
(73) Patentinhaber: N.V. Nutricia, 2712 HM Zoetermeer (NL)
(72) Erfinder: Wolkenstörfer, Reinhold, 91077 Neunkirchen (DE)
(74) Vertreter: von Hellfeld, Axel

(56) Entgegenhaltungen:
- WO-A-95/04564
- WO-A-03/010354
- US-A- 3 070 132
- US-A- 4 105 732
- US-A- 6 060 000
- US-A1- 2001 051 669
- US-A1- 2004 220 534
- US-B1- 6 530 951

## Beschreibung

Die Erfindung betrifft einen Schlauch für die enterale Ernährung, insbesondere einen PEG-Schlauch (PEG = Perkutane Endoskopische Gastrostomie)

Die perkutane endoskopische Gastrostomie ist die Anlage einer Ernährnungssonde mit Hilfe eines Endoskopes durch die Bauchwand in den Magen. Hierfür wird regelmäßig ein Gastroskop eingesetzt, also ein biegsames optisches Instrument mit Objektiv und Okular oder einer elektronischen Bildübertragungseinrichtung. Ein PEG-Schlauch (auch PEG-Sonde genannt), dient der enteralen Ernährung, also einer Ernährung direkt über den Magen-Darm-Trakt. Über den PEG-Schlauch kann Flüssigkeit bzw. flüssige oder dünnbreiige Nahrung verabreicht werden.

Der PEG-Schlauch ist eines der häufigst eingesetzten Mittel zur langfristigen enteralen Ernährung von Patienten, die aufgrund von bestimmten Erkrankungen nicht mehr ausreichend Nahrung über den Mund zu sich nehmen können.

In der Regel erfolgt die Positionierung eines PEG-Schlauches mit Hilfe der sogenannten Fadendurchzugsmethode. Dabei schiebt der Arzt ein Gastroskop über Mund und Speiseröhre in den Magen. Durch Einblasen von Luft entfaltet sich der Magen, sodass er besser einsehbar ist. Sodann wird eine Hohlnadel durch die Bauchwand unter endoskopischer Kontrolle durch die Bauchdecke in den Magen eingeschoben. In der Hohlnadel steckt ein Kunststoffröhrchen. Sodann wird die Hohlnadel herausgezogen und durch das verbleibende Kunststoffröhrchen wird ein Faden in den Magen eingeführt. Dieser Faden kann dann mit einer Zange erfasst werden, die der Arzt über das Gastroskop eingeführt hat. Faden, Zange und Gastroskop werden dann über die Speiseröhre und den Mund herausgezogen. Der PEG-Schlauch kann dann mit dem aus dem Mund herausragenden Fadenende verbunden werden und durch Zug am anderen Fadenende wird die Sonde über Mund und Speiseröhre in den Magen gebracht und teilweise durch die Bauchdecke gezogen. Platten an dem PEG-Schlauch positionieren ihn in Bezug auf die Bauchdecke und den Magen. Sodann kann das herausragende Ende des PEG-Schlauches mit Mitteln zum Zuführen von Nahrung verbunden werden.

Zwar hat sich die perkutane endoskopische Gastrostomie weithin durchgesetzt, jedoch besteht nach wie vor seit langem ein erhebliches Infektionsrisiko, das in den zwei-stelligen Prozentbereich reichen kann. So wird noch im Jahre 2000 allein für die peristaltische Infektion ein Prozentsatz von 5-15% berichtet (C.H. Löser, DMW 125, 805). Insbesondere treten Nekrose und Mykose auf.

Der Stand der Technik kennt verschiedene Versuche, durch PEG-Schläuche verursachte Infektionen zu verhindern. So werden möglichst glatte Oberflächen am PEG-Schlauch eingesetzt, auch hydrophile Oberflächen, sowie Hydrogele, Heparin und Heparioide. Auch monoklonale Antikörper werden bei Kathetern eingesetzt. Studien liegen vor zur Verhinderung von Infektionen bei der PEG mittels Antibiotika.

Allerdings haben Antibiotika auch Nachteile, insbesondere kann die antimikrobielle Wirkung schon nach 24 Stunden nachlassen und die Wirkung ist nicht lokal begrenzt. Auch das Anwendungsspektrum ist begrenzt und der Patient kann Resistenz entwickeln. Auch unter Kostengesichtspunkten sind Antibiotika nachteilig.

Die US 2004/220534 A1 zeigt eine "medizinische Einrichtung", die unter Anderem zur Ernährung eingesetzt werden soll. Eine Parylene-Beschichtung ist dort nicht vorgesehen.

Die WO 95/04564 A, zeigt eine Parylene-Schicht ohne dass eine Metallionen freisetzende Substanz vorgesehen ist.

Die US 2001/051669 A1 beschreibt eine Ernährungssonde, wobei keine Schicht aus Parylene vorgesehen ist.

Die US 6,530,951 B1 beschreibt ein medizinisches Implantat für die perkutane transluminale Angioplastie. Also üblicherweise ein Stent, der durch einen Katheter in eine Ader oder auch z.B. in die Speiseröhre geschoben wird, um die Ader bzw. die Speiseröhre offen zu halten.

Der Erfindung liegt die Aufgabe zugrunde, einen Schlauch für die enterale Ernährung bereitzustellen, bei dem mit einfachen Mitteln die Infektionsgefahr reduziert ist.

Hierfür stellt die Erfindung einen Schlauch für die perkutane endoskopische Gastrostomie gemäß Anspruch 1 bereit. Vorteilhafte Ausgestaltung des Schlauches sind in den abhängigen Ansprüchen beschrieben.

Als Verbindung hierfür sind besonders geeignet Metallsalze, insbesondere Silbersalze, die Metallionen (Silberionen) freisetzen.

Gemäß einer bevorzugten Ausgestaltung der Erfindung liegt die Metallionen freisetzende Verbindung in Form von sogenannten Nanopartikeln vor, also Partikeln mit Abmessungen im Nano-Bereich.

Die Erfindung hat insbesondere folgende Vorteile: ein sehr breitbandiges antimikrobielles Spektrum; eine hohe Aktivität gegen sessile Organismen und Varianten in sehr kleinen Kolonien; eine langanhaltende Aktivität über Wochen, Monate oder sogar Jahre; keine Induktion von Resistenz; keine Förderung von Thrombosegefahr oder Zytotoxizität; keine Änderungen der Materialeigenschaften des Schlauchkunststoffes; sowie geringe Kosten.

Gemäß einer beschriebenen Ausgestaltung ist der Schlauch nicht vollständig in allen Bereichen mit der antiseptischen und/oder antimikrobiellen Substanz versehen, sondern nur in Teilbereichen. Diese Teilbereiche des Schlauches erstrecken sich bevorzugt streifenförmig, insbesondere in Längsrichtung des Schlauches, sodass eine einfache Herstellung mit Extrusionsverfahren ermöglicht ist. Eine bevorzugte Ausgestaltung sieht vor, dass die streifenförmigen Oberflächenbereiche zick-zack-förmig oder wellenförmig sind. Hierdurch wird eine gleichmäßig Abgabe und Verteilung der Metallionen gefördert. Auch können zu diesem Zweck streifenförmige Bereiche mit der antimikrobiellen Verbindung vorgesehen sein, die eine variierende Breite über die Längserstreckung aufweisen oder auch wendelförmig angeordnet sind.

Für eine Förderung der Inspektionsmöglichkeiten kann der Schlauch bevorzugt teilweise mit transparenten Bereichen ausgestaltet sein, die sich in Längsrichtung erstrecken, zum Beispiel indem sein Kunststoffmaterial transparent ist.

Die Erfindung sieht auf der Oberfläche des Schlauches eine Parylene-Beschichtung vor. Diese Parylene-Beschichtung kann auf der Außenfläche und/oder der Innenfläche des Schlauches vorgesehen sein.

Eine weitere Ausgestaltung sieht einen Schlauch aus zum Beispiel Polyurethan oder Silikon vor, der auf der Außenfläche und/oder der Innenfläche mit einer Parylene-Beschichtung versehen ist, wobei über der Parylene-Beschichtung eine weitere Beschichtung aus einem Metallionen freisetzenden Material aufgetragen ist. Bei den Metallionen kann es sich zum Beispiel um Silber-Ionen oder Titan-Ionen handeln.

Bevorzugt wird Parylene C eingesetzt und/oder Parylene N. Es wurde festgestellt, dass für den hier vorgesehenen Einsatz Parylene C eine besonders gute chemische Resistenz aufweist und eine leichte antibakterielle Wirkung. Parylene N hingegen zeichnet sich durch eine besondere Gleitfähigkeit aus. Geeignet ist auch ein Mix aus Parylene C und Parylene N.

Die Erfindung beschreibt auch allgemein Schläuche für die enterale Ernährung, insbesondere Sonden und Katheter, die sich dadurch auszeichnen, dass sie mit einer Beschichtung aus Parylene oder Poly-Parylene versehen sind, wobei die Beschichtung Substanzen enthält, welche antiseptisch oder antimikrobiell wirken, insbesondere durch Freisetzung von Metallionen.

Auch hier, wie bei dem PEG-Schlauch, werden die antiseptisch oder antimikrobiell wirkenden Substanzen vorzugsweise in Form von Nanopartikeln eingebracht oder aufgebracht.

Die Schichtstärke der Beschichtung aus Parylene kann bei allen hier beschriebenen Ausführungsbeispielen vorzugsweise im µm-Bereich liegen, insbesondere im Bereich von 0,2 bis 10 µm, besonders bevorzugt im Bereich von 1 bis 5 µm.

Dabei kann auf die oben bereits angesprochene Parylene-Beschichtung noch eine weitere Beschichtung aufgetragen werden aus einem Material, welches Ag- oder Ti-Ionen freisetzt. Dies gilt sowohl für die äußere Beschichtung des Schlauches als auch seine innere Beschichtung. Techniken zum Aufbringen von derartigen Beschichtungen sind als solche aus anderen Bereichen bekannt, zum Beispiel aus der allgemeinen Technik von Metallbeschichtungen.

Die Konzentration und/oder Schichtstärke der antiseptisch oder antimikrobiell wirkenden, Metallionen freisetzenden Substanzen ist vorzugsweise so bemessen, dass sie insbesondere in den ersten 20 Tagen des Einsatzes wirken, solange die Infektionsgefahr besteht.

Die hier beschriebenen Schläuche bestehen vorzugsweise aus Polyurethan oder Silikon. Es können auch mehrschichtige Schläuche vorgesehen werden, zum Beispiel zweischichtige mit einer etwas dünneren Außenschicht, in welcher die antiseptisch oder antimikrobiell wirkenden Substanzen angeordnet sind, während eine vorzugsweise etwas stärkere innere Schlauchschicht davon frei sein kann. Eine solche mehrschichtige Schlauchkonstruktion ist im Extrusionsverfahren leicht herstellbar. Die Schichtanordnung kann auch umgekehrt als vorstehend beschrieben sein.

Als antiseptisch oder antimikrobiell wirkende, Metallionen freisetzende Substanz dient die Verwendung von ionisiertem Titan, d.h. einer Verbindung, welche Titan in ionischer Form freisetzt.

Nachfolgend wird ein Ausführungsbeispiel der Erfindung anhand der Zeichnung näher erläutert.

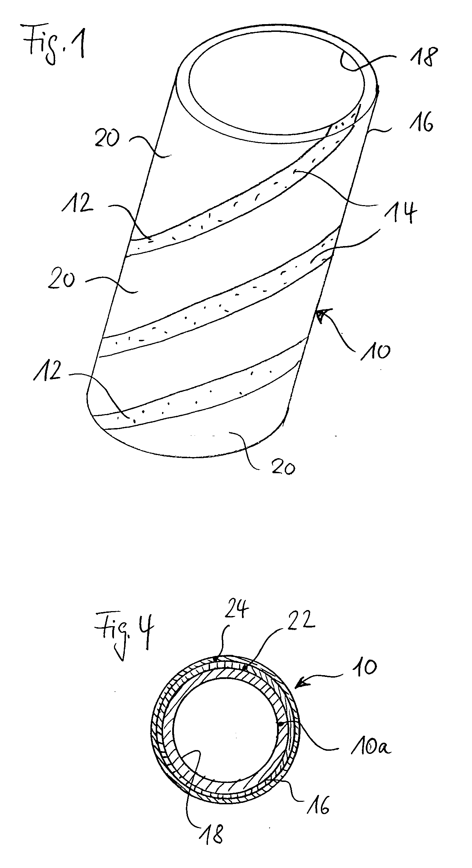

Die Figuren 1, 2 und 3 zeigen schematisch jeweils einen Abschnitt eines PEG-Schlauches. Figur 4 zeigt einen Schnitt durch ein Ausführungsbeispiel eines Schlauches für die enterale Ernährung.

In den Figuren sind einander entsprechende oder funktionsähnliche Komponenten mit den gleichen Bezugszeichen versehen.

Figuren 1, 2 und 3 zeigen einen kurzen Abschnitt eines PEG-Schlauches 10 aus PU. In ausgewählten Bereichen 12 des Schlauches sind Silber-Nano-Partikel 14 eingebracht. Die dazwischenliegenden Bereiche 20 sind frei von solchen Partikeln.

Die mit den silberhaltigen Nanopartikeln versehenen Bereiche 12 erstrecken sich von der Außenfläche 16 des Schlauches 10 bis zur Innenfläche 18.

Die Konzentration der silberhaltigen Nanopartikel ist so, dass sie über längere Zeiträume, insbesondere Zeiträume von vielen Tagen, Wochen oder Monaten kontinuierlich Silberionen mit antibakterieller Wirkung abgeben.

Figur 1 zeigt eine Anordnung der mit der antibakteriellen Verbindung versetzten Bereiche 12, die sich wendelförmig (schraubenlinienförmig) um die Längsachse des Schlauches 10 herum erstrecken.

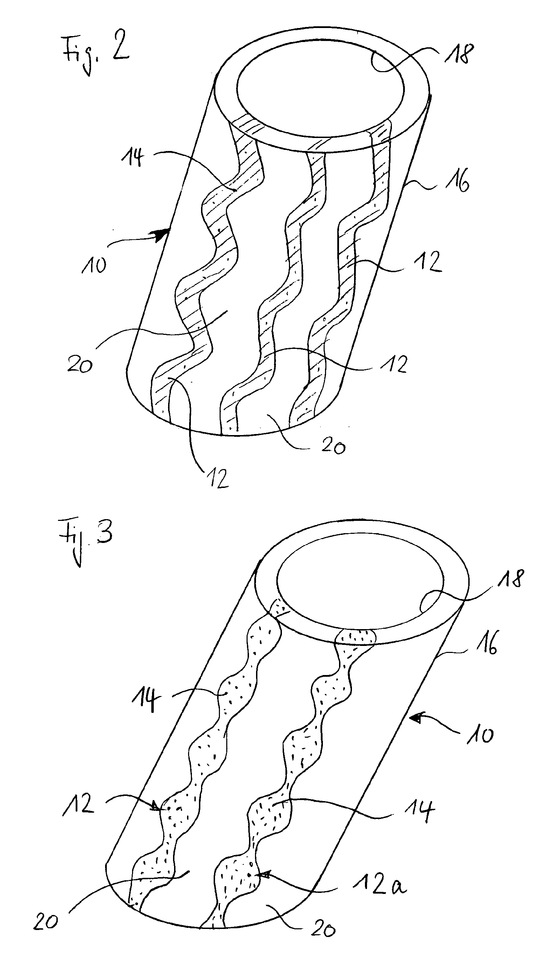

Gemäß Figur 2 erstrecken sich die mit der antibakteriellen Verbindung versetzten Bereiche 12 in Längsrichtung des Schlauches und sind zick-zack-förmig bzw. wellenförmig.

Gemäß Figur 3 sind die entsprechenden Bereiche 12 so gestaltet, dass ihre Breite in Längsrichtung des Schlauches variiert.

Die Darstellungen sind schematisch. Tatsächlich sind die mit der antibakteriellen Substanz versetzten Bereiche so geometrisch gestaltet, dass die wirksamen Metallionen weitestgehend homogen vom Schlauch abgegeben werden, d.h. die verbindungstragenden Bereiche sind so eng beieinanderliegend, dass keine Lücken hinsichtlich der antimikrobiellen Wirkung verbleiben.

Der Schlauch 10 ist mit einer Parylene-Beschichtung versehen, die das Eindringen eines Polyvidon-Jod-Komplex-Wirkstoffes und/oder das Eindringen von Desinfektionsmittel in das Schlauchmaterial verhindert.

Die Innenfläche 18 des Schlauches ist mit einer Schicht versehen, vorzugsweise im Koextrusionsverfahren, die resistent ist gegen Zytostatika, wodurch der Schlauch seine mechanischen Eigenschaften beibehält. Insbesondere bietet Parylene Schutz gegen Zytostatika.

Als Beschichtungsmaterial für den Schlauch kommt Parylene oder Poly-Parylene (Handelsname) in Betracht. Parylene ist ein Polymer aus der Familien Polyp-Xylylene.

Figur 4 zeigt einen Schnitt durch ein Ausführungsbeispiel eines Schlauches 10 für die enterale Ernährung, insbesondere für die PEG. Ein Schlauch 10a aus Polyurethan oder Silikon bildet das Stützgerüst des Schlauches. Auf den Schlauch 10a aufgetragen ist radial außen eine Schicht 22 aus Parylene, insbesondere Parylene C oder Parylene N oder einem Mix aus Parylene C und Parylene N. Über die Schicht 22 ist eine weitere Schicht 24 aufgebracht aus einem antimikrobiell wirkenden, Metallionen freisetzenden Material, wobei die Metallionen bevorzugt Silberionen oder Titanionen sind.

Das Ausführungsbeispiel gemäß Figur 4 kann dahingehend ergänzt werden, dass eine analoge Beschichtung auf der Innenfläche 18 des Schlauches 10a aufgebracht wird, also auf die Innenfläche 18 zunächst eine Beschichtung (analog der Schicht 22) aus Parylene C und/oder Parylene N und innenseitig darauf dann eine weitere Beschichtung (analog der Beschichtung 24) aus einem Metallionen freisetzenden Material.

In Figur 4 sind die Schichtstärken nicht maßstabsgetreu; vielmehr sind zur vereinfachten Darstellung die äußeren Schichten verstärkt gezeichnet.

In der vorstehenden Beschreibung und der Zeichnung sind allgemein Schläuche für die enterale Ernährung dargestellt, insbesondere PEG-Schläuche. Sämtliche beschriebenen Einzelheiten, Merkmale und Maßnahmen sind sowohl für den Einsatz bei PEG-Schläuchen als auch ganz allgemein für den Einsatz bei Schläuchen für die enterale Ernährung geeignet und einsetzbar.

## Patentansprüche

1. Perkutane Endoskopische Gastrostomie (PEG)-Schlauch mit einer antiseptisch oder antimikrobiell wirkenden, Metallionen freisetzenden Verbindung zumindest auf oder in Oberflächenbereichen (12) des Schlauches (10), **dadurch gekennzeichnet, dass** der Schlauch (10) eine Parylene-Beschichtung aufweist, in die eine antiseptisch oder antimikrobiell wirkende, Metallionen freisetzende Verbindung eingebracht ist, oder dass auf die Parylene-Beschichtung eine Schicht (24) aus einer antiseptisch oder antimikrobiell wirkenden, Metallionen freisetzenden Verbindung aufgebracht ist.

2. PEG-Schlauch nach Anspruch 1, **dadurch gekennzeichnet, dass** die Parylene-Beschichtung aus Parylene N und/oder Parylene C ist.

3. PEG-Schlauch nach Anspruch 1 oder 2, wobei die Metallionen Silber- oder Titanionen sind.

4. PEG-Schlauch nach einem der vorhergehenden Ansprüche, **gekennzeichnet durch** eine Parylene-Beschichtung auf der Außenfläche (16) und/oder Innenfläche (18) des Schlauches (10).

5. PEG-Schlauch nach einem der vorhergehenden Ansprüche, **dadurch gekennzeichnet, dass** der Schlauch (10) zumindest teilweise transparent ist.

6. PEG-Schlauch nach einem der vorhergehenden Ansprüche, **dadurch gekennzeichnet, dass** der Schlauch (10) zumindest teilweise opak ist.

7. PEG-Schlauch nach einem der vorhergehenden Ansprüche, wobei die antiseptisch oder antimikrobiell wirkende, Metallionen freisetzende Verbindung in Form von Nanopartikeln vorliegt.

8. PEG-Schlauch nach einem der vorhergehenden Ansprüche aus Polyurethan oder Silikon.

## Claims

1. Percutaneous endoscopic gastrostomy (PEG) tube having an antiseptically or antimicrobially acting, metal ions releasing compound at least on or in surface regions (12) of the tube (10), **characterized in that** the tube (10) has a Parylene coating into which an antiseptically or antimicrobially acting, metal ions releasing compound is introduced, or that a layer (24) of an antiseptically or antimicrobially acting, metal ions releasing compound is applied to the Parylene coating.

2. PEG tube according to claim 1, **characterized in that** the Parylene coating is of Parylene N and/or Parylene C.

3. PEG tube according to claim 1 or 2, wherein the metal ions are silver ions or titanium ions.

4. PEG tube according to any one of the preceding claims, **characterized by** a Parylene coating on the outer surface (16) and/or inner surface (18) of the tube (10).

5. PEG tube according to any one of the preceding claims, **characterized in that** the tube (10) is at least partially transparent.

6. PEG tube according to any one of the preceding claims, **characterized in that** the tube (10) is at least partially opaque.

7. PEG tube according to any one of the preceding claims, wherein the antiseptically or antimicrobially acting, metal ions releasing compound is provided in the form of nanoparticles.

8. PEG tube according to any one of the preceding claims, made of polyurethane or silicone.

## Revendications

1. Tuyau flexible pour la gastrostomie endoscopique percutanée (GPE), comprenant un composé antiseptique ou antimicrobien, libérant des ions métalliques, au moins sur ou dans des zones de surface (12) du tuyau flexible (10), **caractérisé en ce que** le tuyau flexible (10) présente un revêtement en parylène dans lequel un composé antiseptique ou antimicrobien, libérant des ions métalliques, est introduit, ou **en ce qu'**une couche (24) d'un composé antiseptique ou antimicrobien, libérant des ions métalliques, est appliquée sur le revêtement en parylène.

2. Tuyau flexible pour la GEP selon la revendication 1, **caractérisé en ce que** le revêtement en parylène est en parylène N et/ou en parylène C.

3. Tuyau flexible pour la GEP selon les revendications 1 ou 2, dans lequel les ions métalliques sont des ions argent ou titane.

4. Tuyau flexible pour la GEP selon l'une des revendications précédentes, **caractérisé par** un revêtement en parylène sur la surface extérieure (16) et/ou la surface intérieure (18) du tuyau flexible (10).

5. Tuyau flexible pour la GEP selon l'une des revendications précédentes, **caractérisé en ce que** le tuyau flexible (10) est au moins partiellement transparent.

6. Tuyau flexible pour la GEP selon l'une des revendications précédentes, **caractérisé en ce que** le tuyau flexible (10) est au moins partiellement opaque.

7. Tuyau flexible pour la GEP selon l'une des revendications précédentes, dans lequel le composé antiseptique ou antimicrobien, libérant des ions métalliques, est présent sous la forme de nanoparticules.

8. Tuyau flexible pour la GEP selon l'une des revendications précédentes, réalisé en polyuréthane ou silicone.
